# EUROPÄISCHE PATENTSCHRIFT

(11) **EP 1 150 609 B1**
(45) Veröffentlichungstag und Bekanntmachung des Hinweises auf die Patenterteilung: **20.07.2005**
(21) Anmeldenummer: 00912352.2
(22) Anmeldetag: 03.02.2000
(51) Int. Cl.: A61B 5/20, B01L 3/00

(54) **EINRICHTUNG ZUR AUFNAHME UND KONTROLLE AUSGESCHIEDENEN URINS**
DEVICE FOR RECEIVING AND CONTROLLING VOIDED URINE
DISPOSITIF PERMETTANT DE RECUEILLIR ET D'ANALYSER L'URINE

(30) Priorität: 04.02.1999 DE 19904556
(43) Veröffentlichungstag der Anmeldung: 07.11.2001
(73) Patentinhaber: RAHE, Martin, D-32609 Hüllhorst (DE)
(72) Erfinder: RAHE, Martin, D-32609 Hüllhorst (DE)
(74) Vertreter: Czybulka, Uwe
(86) Internationale Anmeldenummer: PCT/DE2000/000334
(87) Internationale Veröffentlichungsnummer: WO 2000/045705

(56) Entgegenhaltungen:
- WO-A-92/15863
- WO-A-95/19845

## Beschreibung

Die vorliegende Erfindung betrifft eine Einrichtung zur Aufnahme und Kontrolle ausgeschiedenen Urins und insbesondere unkontrollierbar ausgeschiedenen Urins gemäß dem Oberbegriff des Patentanspruchs 1.

Eine derartige Vorrichtung ist aus der EP 0 560 099 A2 bekannt: Sie weist eine den Innenraum der Vorrichtung wenigstens teilweise definierende und umhüllende Wandung auf, die wenigstens einen die zu untersuchende Flüssigkeit bzw. den Urin über einen vorbestimmten Zeitraum in das Vorrichtungsinnere einlassenden Durchlass aufweist. Außerdem ist eine die Flüssigkeit bzw. den Urin in das Vorrichtungsinnere fördende Einrichtung sowie eine den Flüssigkeitseinlauf stoppende Einrichtung vorgesehen. Die Kontrolle der Beschaffenheit der zu untersuchenden Flüssigkeit wird mittels geeigneter Indikatorkarten (Check-up-Karten) durchgeführt, wobei die konstruktionsbedingt mit einem gewissen Aufwand verbundene Ablesbarkeit der Resultate einen Nachteil darstellt, da diese von der der zu untersuchenden Flüssigkkeit abgewandten Seite der Vorrichtung nicht sichtbar sind.

Die EP 0 438 482 B1 offenbart ebenfalls eine solche Einrichtung zur Aufnahme und Kontrolle in diesem Falle unkontrollierbar ausgeschiedenen Urins; die Einrichtung besteht aus einer kleinen Messzelle in Form einer "Kontrollkarte", die in einem durchsichtigen Auffangbeutel plaziert ist, in den der Urin durch einen Einleitschlauch gelangt. Auf der Kontrollkarte sind zur Kontrolle des aufgefangenen Urins hinsichtlich sich anbahnender und bestehender Keiminfektionen urinexponierte Indikatoren vorgesehen, die z. B. auf pH-, Nitrit-, Leukozyten- und Elektrolytwerte des Urins ansprechen. Die Kontrollkarte ist auf der urinexponierten Seite mit einer Membran überzogen, die die Urinaufnahme verlangsamt, d. h. den Urin nur langsam zu den Indikatoren durchdringen lässt, und damit "Fehlmessungen" allein durch den Vorlaufurin mit seiner üblicherweise hohen Keimkonzentration verhindert.

Zwischen Membran und Indikatoren ist noch ein aufquellendes Material angeordnet, das nach Eintritt des Urins in die Messzelle aufquillt, gegen die Membran drückt und diese nach einer gewissen Zeit verschließt. Hiermit wird einer Ausschwemmung der Indikatorsubstanzen entgegengewirkt.

Bei dieser bekannten Messzelle werden die Indikatoren durch die durchsichtige Außenfolie bzw. ein Sichtfenster von der der Membran gegenüberliegenden Seite betrachtet, da auf der anderen Seite die Indikatoren durch das aufquellende Material verdeckt sind. Dieses Material muss direkt an der "Rückseite" der Indikatoren anliegen, so dass diese durch den Urin von der Rückseite aus erst langsam durchtränkt werden müssen, ehe ein Farbumschlag erfolgt und durch das Sichtfenster wahrgenommen werden kann.

Auf dem Markt sind für diese bekannte Einrichtung eigentlich gut verwendbare Indikatoren erhältlich, die jedoch aus fertigungstechnischen Gründen und aufgrund ihrer ansonsten unterschiedlichen Anwendung, z. B. direktes Benetzen mit Urin zur Feststellung etwaiger Krankheiten, auf einem zumindest leicht opaken, annähernd weißen Kunststoffstreifen aufgebracht sind. Wenn diese Indikatoren in der bekannten Einrichtung eingesetzt würden, müssten die Indikatoren mit ihrer Oberfläche gegen das aufquellende Material plaziert werden, so dass der Farbumschlag durch den Kunststoffstreifen betrachtet werden muss. Durch das wenig transparente Material des Streifens ist der Farbumschlag jedoch nur undeutlich feststellbar, insbesondere ist auch die Intensität des Farbumschlages schlecht wahrnehmbar.

Eine Einrichtung gemäß dem Oberbegriff des Anspruchs 1 ist aus der WO 92/75863 bekannt.

Der vorliegenden Erfindung liegt die Aufgabe zugrunde, die bekannte Einrichtung dahingehend zu verbessern, dass die Indikatoren leicht ablesbar sind und eine zuverlässige, eindeutige Aussage über die Beschaffenheit des Urins ermöglicht wird. Darüber hinaus soll es möglich sein, die Einrichtung unterschiedlichen Einsatzbereichen anzupassen.

Diese Aufgabe wird gemäß der Erfindung durch die Merkmale des Patentanspruches 1 gelöst. Weitere Ausgestaltungen der Erfindung gehen aus den Unteransprüchen hervor.

Demnach wird mit der vorliegenden Erfindung vorgeschlagen, handelsübliche, auf einem im wesentlichen undurchsichtigen Träger angeordnete Indikatoren zu verwenden, die durch ein Sichtfenster der Messzelle wahrnehmbar sind. Um den Urin von der auf der gegenüber liegenden Seite der Messzelle gelegenen Zuflussöffnung auf die Indikatoren zu leiten, ist ein durch Kapillarität zum Transportieren von Flüssigkeiten geeignetes Flüssigkeitstransportmittel, z. B. Fließpapier, vorgesehen.

Wie bei der bekannten Messzelle kann auch hier ein aufquellendes Material, z. B. ein Quellvlies vorgesehen sein, das zwischen Indikatorträger und Zuflussöffnung angeordnet ist, das bei der Aufnahme von Urin aufquillt und die Zuflussöffnung der Messzelle nach wenigen Minuten verschließt.

Im Rahmen einer weiteren Variante der erfindungsgemäßen Einrichtung, die insbesondere in aufsaugenden Inkontinenzmitteln (Windeln) eingesetzt werden soll, wird auf ein separates Quellkissen verzichtet und der Indikatorträger mit Fließpapier oder einem ähnlichen kapillaren Mittel umhüllt. Ein Abdichten des Systems ist in diesem Fall von untergeordneter Bedeutung. Das Fließpapier ermöglicht in diesem Falle überhaupt erst das Eindringen der Flüssigkeit in die Meßzelle, da bei den verwendeten kleinen Zuflussöffnungen der Luftdruck im Inneren der Messzelle ein Eindringen von Flüssigkeit verhinderte, wenn nicht durch den Kapillardruck im Fließpapier Flüssigkeit angesaugt würde.

Die Leucozytenmenge pro Volumen wird wie folgt ermittelt: Die Indikatoren enthalten Indoxylester, der durch Granulozyten-Esterasen gespalten wird. Folglich ist es möglich, durch die Granulozyten-Esterasen-Konzentration auf die Leukozytenmenge pro Volumen zu schließen. Das freigesetzte Spaltprodukt Indoxyl reagiert mit dem Diozoniumsalz im Indikator zu einem violetten Farbstoff. Je nach Konzentration entsteht ein Farbumschlag vom beige nach violett.

Um die Anzeige der Leucozytenmenge, insbesondere bei einer sehr geringen Leucozyten-Konzentration zu verbessern, wird im Rahmen der Erfindung vorgeschlagen, neben den Indikatoren auch das verwendete Fließpapier mit Indoxylester zu imprägnieren. Somit wird durch die Granulozyten-Esterasen durch die höhere Indoxylester-Konzentration mehr Indoxyl freigesetzt, wodurch eine dunklere Färbung entsteht. Ein dunkler Farbumschlag ist qualitativ eindeutiger sowie wesentlich stabiler als ein heller, wodurch die Anzeige qualitativ verbessert wird.

Um die Zuverlässigkeit des Systems weiter zu erhöhen, wird außerdem vorgeschlagen, das Fließpapier durchlässiger auszubilden, um ein Zurückhalten der relativ großen Leukozyten vom Fließpapier zu vermeiden. Durch eine rillenförmige Prägung des Fließpapiers wird dieses durchlässiger.

Des weiteren wird vorgeschlagen, das Fließpapier mit Substanzen zu präparieren, welche den Reaktionsablauf der Indikatoren hinsichtlich Farbstabilisierung, Empfindlichkeit, Fließeigenschaften usw. entsprechend positiv beeinflussen. Dadurch wird verhindert, dass nach einer längeren Zeit (15-20 Minuten) Wechselwirkungen zwischen den in der Meßzelle enthaltenen Indikatoren auftreten. In diesem Zusammenhang kann ein Indikatorstreifen durch ein entsprechend präpariertes Papier ausgetauscht werden oder ein zusätzliches getränktes Papier in den Teststreifen integriert werden.

Die unterschiedlichen Indikatoren benötigen für eine bessere Funktionsweise unterschiedliche Mengen an Testflüssigkeit. Folglich wird vorgeschlagen, die Fließbahnen der Testflüssigkeit unterschiedlich breit zu gestalten, um die Mengen der Testflüssigkeit zu steuern.

Die Erfindung ist im folgenden anhand der Zeichnung näher erläutert. In dieser stellen dar:
- Fig. 1: eine schematische Querschnittsansicht eines ersten Ausführungsbeispieles der Einrichtung gemäß der Erfindung;
- Fig. 2: eine schematische Querschnittsansicht eines zweiten Ausführungsbeispieles der Einrichtung gemäß der Erfindung; und
- Fig. 3: eine Darstellung der Fließpapier-Rückseite gemäß einer vorteilhaften Ausführungsform der Erfindung.

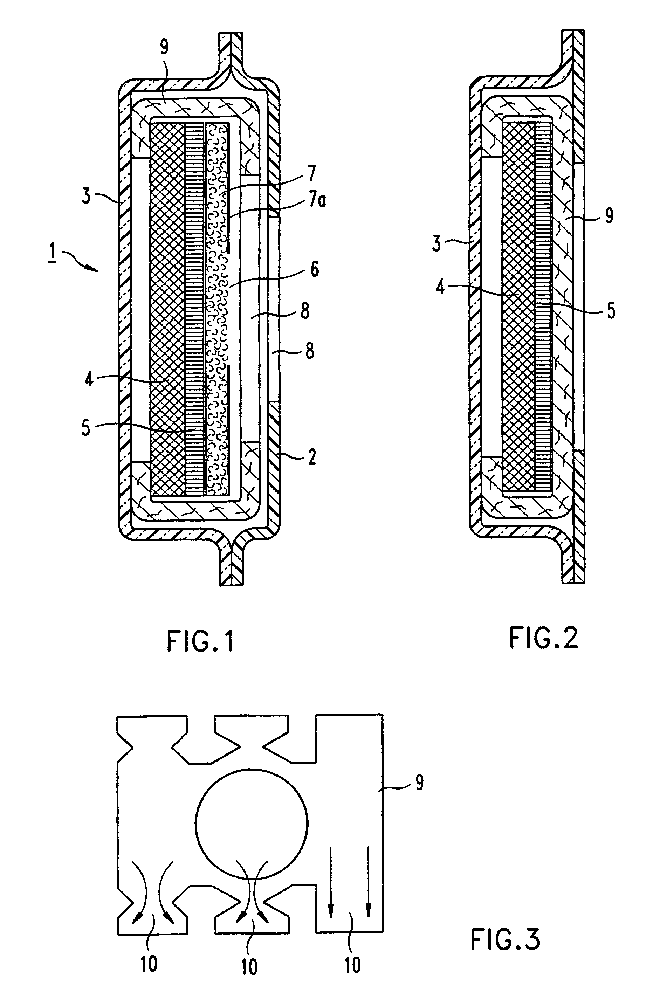

Gemäß Figur 1 enthält die Einrichtung zur Aufnahme und Kontrolle unkontrollierbar ausgeschiedenen Urins eine geschlossene flache Messzelle 1, vorzugsweise aus PP-Folie, mit einer rückseitigen Stützfolie 2 und einer einen transparenten Bereich bildenden durchsichtigen Frontfolie, die als Sichtfenster 3 dient. Hinter dem Sichtfenster 3 sind Indikatoren 4 auf einem Indikatorträger 5 aufgetragen. In der hinteren Stützfolie ist eine einige Millimeter große Zuflussöffnung 6 vorgesehen.

Außerdem ist in der Messzelle 1 aufquellendes Material, vorzugsweise ein Quellkissen 7, das mit einer Folie 7a kaschiert ist, mit einer einige Millimeter großen Zuflussöffnung 8 angeordnet. Die Zuflussöffnungen 6 bzw. 8 sind im wesentlichen unmittelbar hintereinander angeordnet.

Ein Flüssigkeitstransportmittel 9, das bei dieser dieser Ausführungsform ein Fließpapierbogen ist, das durch seine Kapillarität zum Transportieren von Flüssigkeiten geeignet ist, umgibt das Quellkissen, den Indikatorträger und die Indikatoren, wobei der eine Rand des Fließpapieres nahe der Zuflussöffnungen 6, 8 gelegen und der andere Rand einen Randbereich aller Indikatoren bedeckt.

Die Funktionsweise der Einrichtung ist wie folgt: Der Urin gelangt über die Zuflussöffnungen 6, 8 in das Quellkissen 7 bzw. Quellvlies, welches dadurch aufquillt. Mittels des als Flüssigkeitstransportmittel 9 eingesetzten kapillaren Fließpapiers wird gleichzeitig eine geringe Menge Urin angesaugt, die dann um das Quellkissen 7 und den undurchlässigen Indikatorträger 5 herum zu den Indikatoren 4 befördert wird. Das Volumen des Quellkissens 7 nimmt durch den Quellprozess ständig zu, wodurch die Meßzelle nach wenigen Minuten verschlossen wird.

Das als Flüssigkeitstransportmittel 9 eingesetzte Fließpapier kann natürlich durch ein anderes Mittel ersetzt werden, z. B. einen Docht oder ein geeignetes Flüssigkeitstransportmittel aus nicht zellulosem Material.

Figur 2 zeigt eine weitere Variante der Erfindung, die insbesondere in aufsaugenden Inkontinenzmitteln (Windeln) eingesetzt werden soll. Hierbei wird im Gegensatz zu der eingangs beschriebenen Ausführungsform kein separates Quellkissen verwendet, da ein Abdichten des Systems in diesem Fall von untergeordneter Bedeutung ist. Das Fließpapier erstreckt sich über einen weiteren Bereich und umhüllt im wesenlichen die gesamte Rückseite des Indikatorträgers 5. Diese Konstruktion hat eine sehr kurze Ansprechzeit und benötigt für die Indikatoren nur eine geringe Urinmenge. In Abwandlung dieses Ausführungsbeispieles kann auch ein Quellkissen verwendet werden, das um den Indikatorträger herumgeführt und mit einem Randbereich der Indikatoren verbunden ist. Quellkissen und Flüssigkeitstranssportmittel sind somit das gleiche Teil.

Beide beschriebenen Ausführungsformen können erfindungsgemäß zur Verbesserung der Anzeige der Leucozytenmenge, insbesondere bei einer sehr geringen Leucozyten-Konzentration mit mit Indoxylester imprägniertem Fließpapier versehen werden. Dadurch wird, wie eingangs erläutert, durch die höhere Indoxylester-Konzentration mehr Indoxyl freigesetzt, wodurch eine dunklere qualitativ eindeutigere Färbung entsteht.

Eine Übertragung des Prinzips, das Fließpapier mit Substanzen zu präparieren, welche den Reaktionsablauf der Indikatoren hinsichtlich Farbstabilisierung, Empfindlichkeit, Fließeigenschaften positiv beeinflussen ist im Rahmen einer weiteren Variante der Erfindung realisierbar.

Gemäß Figur 3 sind die Fließbahnen 10 der Testflüssigkeit unterschiedlich breit ausgebildet, um die Mengen der Testflüssigkeit zu steuern, da die unterschiedlichen Indikatoren für eine bessere Funktionsweise unterschiedliche Mengen an Testflüssigkeit benötigen. So ist z.B. die rechte Fließbahn breiter als die beiden anderen, um eine hohe Zuverlässigkeit und Durchlässigkeit zu gewährleisten.

## Patentansprüche

1. Einrichtung zur Aufnahme und Kontrolle ausgeschiedenen Urins in Form einer Messzelle (1), die zumindest eine Zuflussöffnung (6) und in ihrem Inneren Indikatoren (4) aufweist und die Messzelle (1) zumindest einen, ein Sichtfenster (3) bildenden transparenten Bereich aufweist, durch den die Indikatoren (4) sichtbar sind, wobei die Indikatoren (4) auf einer dem Sichtfenster (3) zugewandten Seite eines Indikatorträgers (5) gelegen sind, **dadurch gekennzeichnet, dass** ein durch seine Kapillarität zum Transportieren von Flüssigkeiten geeignetes Flüssigkeitstransportmittel (9) von der Zuflussöffnung (6) ausgehend den Indikatorträger (5) umgreift und mit zumindest einem Bereich eines jeden Indikators (4) auf der dem Sichtfenster (3) zugewandten Seite verbunden ist.

2. Einrichtung nach Anspruch 1, **dadurch gekennzeichnet, dass** ein aufquellendes Material zwischen Indikatorträger (5) und Zuflussöffnung (6) gelegen ist.

3. Einrichtung nach Anspruch 2, **dadurch gekennzeichnet, dass** das aufquellende Material ein Quellkissen (7) aus einem Quellvlies ist.

4. Einrichtung nach Anspruch 3, **dadurch gekennzeichnet, dass** das Quellkissen (7) mit einer Folie (7a) kaschiert ist und die Folie eine Zuflussöffnung (8) aufweist, wobei die Zuflussöffnungen (6, 8) der Messzelle (1) und des Quellkissens (7) unmittelbar hintereinander angeordnet sind.

5. Einrichtung nach einem der vorhergehenden Ansprüche, **dadurch gekennzeichnet, dass** das Flüssigkeitstransportmittel (9) bis in den Bereich der Zuflussöffnug (6) reicht.

6. Einrichtung nach einem der vorhergehenden Ansprüche, **dadurch gekennzeichnet, dass** das Flüssigkeitstransportmittel (9) die Rückseite des Indikatorträgers (5) umhüllt.

7. Einrichtung nach einem der vorhergehenden Ansprüche, **dadurch gekennzeichnet, dass** das Flüssigkeitstransportmittel (9) Teil des aufquellenden Materiales ist.

8. Einrichtung nach Anspruch 6, **dadurch gekennzeichnet, dass** das Flüssigkeitstransportmittel (9) Fließpapier ist.

9. Einrichtung nach einem der Ansprüche 1 bis 5, **dadurch gekennzeichnet, dass** das Flüssigkeitstransportmittel (9) aus nicht-zellulosem Material ist.

10. Einrichtung nach einem der vorangehenden Ansprüche, **dadurch gekennzeichnet, dass** zur Verbesserung der Anzeige der Leukozytenmenge pro Volumen das Flüssigkeitstransportmittel (9) mit Indoxylester imprägniert ist, so dass durch die höhere Indoxylester-Konzentration mehr Indoxyl freigesetzt wird und eine dunklere und qualitativ eindeutigere Färbung des auf Leukozytenwerte ansprechenden Indikators (4) entsteht.

11. Einrichtung nach einem der vorangehenden Ansprüche, **dadurch gekennzeichnet, dass** das Flüssigkeitstransportmittel (9) mit Substanzen präpariert ist, welche den Reaktionsablauf der Indikatoren (4) hinsichtlich Farbstabilisierung, Empfindlichkeit, Fließeigenschaften etc. positiv beeinflussen.

12. Einrichtung nach einem der vorangehenden Ansprüche, **dadurch gekennzeichnet, dass** auf dem Flüssigkeitstransportmittel (9) unterschiedlich breite Fließbahnen (10) für den Urin ausgebildet sind, um die Mengen des Urins derart zu steuern, dass die unterschiedlichen Indikatoren (4) die für eine optimale Funktionsweise erforderlichen Mengen an Testflüssigkeit erhalten.

13. Einrichtung nach einem der vorangehenden Ansprüche, **dadurch gekennzeichnet, dass** die Meßzelle aus PP-Folie ausgebildet ist.

## Claims

1. A device for receiving and checking excreted urine in the form of a measuring cell (1) which has at least one, feed flow opening (6) and in its interior indicators (4) and the measuring cell (1) has at least one transparent region which forms a viewing window (3) and through which the indicators (4) are visible, wherein the indicators (4) are disposed on a side of an indicator carrier (5), that is towards the viewing window (3), **characterised in that** a liquid transport means (9) which by virtue of its capillarity is suitable for transporting liquids extends around the indicator carrier (5) from the feed flow opening (6) and is connected to at least one region of each indicator (4) on the side that is towards the viewing window (3).

2. A device according to claim 1 **characterised in that** a swelling material is disposed between the indicator carrier (5) and the feed flow opening (6).

3. A device according to claim 2 **characterised in that** the swelling material is a swelling pad (7) of a swelling non-woven material.

4. A device according to claim 3 **characterised in that** the swelling pad (7) is covered with a film (7a) and the film has a feed flow opening (8), wherein the feed flow openings (6, 8) of the measuring cell (1) and the swelling pad (7) are arranged directly one behind the other.

5. A device according to one of the preceding claims **characterised in that** the liquid transport means (9) extends into the region of the feed flow opening (6).

6. A device according to one of the preceding claims **characterised in that** the liquid transport means (9) encases the rear side of the indicator carrier (5).

7. A device according to one of the preceding claims **characterised in that** the liquid transport means (9) is part of the swelling material.

8. A device according to claim 6 **characterised in that** the liquid transport means (9) is blotting paper.

9. A device according to one of claims 1 to 5 **characterised in that** the liquid transport means (9) is of non-cellulose material.

10. A device according to one of the preceding claims **characterised in that** to improve the indication of the amount of leucocytes per volume the liquid transport means (9) is impregnated with indoxyl ester so that due to the higher indoxyl ester concentration more indoxyl is liberated and darker and qualitatively clearer coloration of the indicator (4) responsive to leucocyte values occurs.

11. A device according to one of the preceding claims **characterised in that** the liquid transport means (9) is prepared with substances which positively influence the course of the reaction of the indicators (4) in respect of colour stabilisation, sensitivity, flow properties etc.

12. A device according to one of the preceding claims **characterised in that** provided on the liquid transport means (9) are flow paths (10) of different widths for the urine in order to control the amounts of urine in such a way that the different indicators (4) receive the amounts of test liquid, which are required for optimum operation.

13. A device according to one of the preceding claims **characterised in that** the measuring cell is formed from PP-film.

## Revendications

1. Dispositif permettant de recueillir et de contrôler de l'urine éliminée, sous forme d'une cellule de mesure (1) qui présente au moins un orifice d'arrivée de liquide (6) et des indicateurs (4) à l'intérieur de celle-ci, et la cellule de mesure (1) présente au moins une zone transparente formant une fenêtre d'inspection (3) à travers laquelle les indicateurs sont visibles, les indicateurs (4) étant situés sur une face du support d'indicateur (5) qui est tournée vers la fenêtre d'inspection (3), **caractérisé en ce qu'**un moyen de transport de liquide (9) approprié au transport de liquides en raison de sa capillarité entoure le support d'indicateur (5) en partant de l'orifice d'arrivée de liquide (6) et est relié avec au moins une zone de chaque indicateur (4) sur la face tournée vers la fenêtre d'inspection (3).

2. Dispositif selon la revendication 1, **caractérisé en ce qu'**un matériau gonflant est situé entre le support d'indicateur (5) et l'orifice d'arrivée de liquide (6).

3. Dispositif selon la revendication 2, **caractérisé en ce que** le matériau gonflant est un coussin gonflant (7) en non-tissé gonflant.

4. Dispositif selon la revendication 3, **caractérisé en ce que** le coussin gonflant (7) est recouvert d'une feuille (7a) et la feuille présente un orifice d'arrivée de liquide (8), les orifices d'arrivées de liquide (6, 8) de la cellule de mesure (1) et du coussin gonflant (7) étant agencés directement l'un derrière l'autre.

5. Dispositif selon l'une des revendications précédentes, **caractérisé en ce que** le moyen de transport de liquide (9) va jusque dans la région de l'orifice d'arrivée de liquide (6).

6. Dispositif selon l'une des revendications précédentes, **caractérisé en ce que** le moyen de transport de liquide (9) enveloppe la face postérieure du support d'indicateur (5).

7. Dispositif selon l'une des revendications précédentes, **caractérisé en ce que** le moyen de transport de liquide (9) fait partie du matériau gonflant.

8. Dispositif selon l'une des revendications précédentes, **caractérisé en ce que** le moyen de transport de liquide (9) est du papier absorbant.

9. Dispositif selon l'une des revendications 1 à 5, **caractérisé en ce que** le moyen de transport de liquide (9) est en matériau non cellulosique.

10. Dispositif selon l'une des revendications précédentes, **caractérisé en ce que** pour améliorer l'affichage de la quantité de leucocytes par volume, le moyen de transport de liquide (9) est imprégné d'un ester indoxylique, de sorte que par la plus forte concentration d'ester indoxylique, il est dégagé plus d'indoxyle et il se produit une coloration plus sombre et plus nette du point de vue qualitatif de l'indicateur (4) réagissant aux taux de leucocytes.

11. Dispositif selon l'une des revendications précédentes, **caractérisé en ce que** le moyen de transport de liquide (9) est préparé avec des substances qui influencent positivement le déroulement réactif des indicateurs (4) du point de vue de la stabilisation de la coloration, de la sensibilité, des propriétés d'écoulement, etc.

12. Dispositif selon l'une des revendications précédentes, **caractérisé en ce que** sur le moyen de transport de liquide (9) sont réalisées des bandes d'écoulement (10) de différentes largeurs pour l'urine, pour commander les quantités d'urine de telle sorte que les différents indicateurs (4) reçoivent les quantités de liquide d'essai nécessaires pour un fonctionnement optimal.

13. Dispositif selon l'une des revendications précédentes, **caractérisé en ce que** la cellule de mesure est réalisée en une feuille de polypropylène.
